(19) Europäisches Patentamt
European Patent Office
Office européen des brevets

(11) Numéro de publication : **0 390 682 B1**

(12) # FASCICULE DE BREVET EUROPEEN

(45) Date de publication du fascicule du brevet : **22.06.94 Bulletin 94/25**

(51) Int. Cl.$^5$ : **C07C 309/24,** A61K 7/06, A61K 7/42, A61K 31/185

(21) Numéro de dépôt : **90400847.1**

(22) Date de dépôt : **28.03.90**

(54) **Compositions cosmétiques et pharmaceutiques contenant des dérivés hydrophiles du benzylidène camphre et nouveaux dérivés sulfonés hydrophilesdu benzylidène camphre.**

(30) Priorité : **31.03.89 FR 8904298**

(43) Date de publication de la demande : **03.10.90 Bulletin 90/40**

(45) Mention de la délivrance du brevet : **22.06.94 Bulletin 94/25**

(84) Etats contractants désignés : **AT BE CH DE DK FR GB IT LI NL SE**

(56) Documents cités :
**GB-A- 2 025 957**
**GB-A- 2 121 801**
**GB-A- 2 150 436**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Lagrange, Alain**
**29 rue Auguste Renoir**
**F-78400 Chatou (FR)**
Inventeur : **Lang, Gérard**
**44 avenue Lacour**
**F-95210 Saint-Gratien (FR)**
Inventeur : **Deflandre, André**
**Route de Manon**
**F-60560 Orry-la-Ville (FR)**
Inventeur : **Luppi, Bernadette**
**43, rue Berlioz**
**F-93270 Sevran (FR)**

(74) Mandataire : **Casalonga, Axel et al**
**BUREAU D.A. CASALONGA - JOSSE**
**Morassistrasse 8**
**D-80469 München (DE)**

EP 0 390 682 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des compositions cosmétiques à usage quotidien ou antisolaire et des compositions pharmaceutiques pour le traitement des inflammations et allergies cutanées contenant des dérivés hydrophiles du benzylidène camphre ainsi que de nouveaux dérivés sulfonés hydrophiles du benzylidène camphre.

Il est bien connu que la peau est sensible aux radiations solaires qui peuvent provoquer un banal coup de soleil ou érythème mais aussi des brûlures plus ou moins accentuées.

Cependant, les radiations solaires ont également d'autres effets néfastes tels qu'une perte d'élasticité de la peau et l'apparition de rides conduisant à un vieillissement prématuré. On peut même aussi observer parfois des dermatoses.

Il est également souhaitable d'assurer aux cheveux une bonne protection contre la dégradation photochimique afin d'éviter un changement de nuance, une décoloration ou une dégradation des propriétés mécaniques.

On sait par ailleurs que les constituants entrant dans les préparations cosmétiques ne possèdent pas toujours une stabilité suffisante à la lumière et se dégradent sous l'action des radiations lumineuses.

Il est bien connu que la partie la plus dangereuse du rayonnement solaire est constituée par les radiations ultraviolettes de longueurs d'onde inférieures à 400 nm. On sait aussi que, de par l'existence de la couche d'ozone de l'atmosphère terrestre qui absorbe une partie du rayonnement solaire, la limite inférieure du rayonnement ultraviolet atteignant la surface de la terre se situe aux environs de 280 nm.

Par conséquent, il paraît souhaitable de disposer de composés susceptibles d'absorber les radiations ultraviolettes dans une large bande de longueurs d'onde allant de 280 à 400 nm, c'est-à-dire aussi bien les rayons UV-B de longueurs d'onde comprises entre 280 et 320 nm jouant un rôle prépondérant dans la production de l'érythème solaire, que les rayons UV-A de longueurs d'onde comprises entre 320 et 400 nm provoquant le brunissement de la peau, mais aussi son vieillissement et favorisant le déclenchement de la réaction érythémateuse ou amplifiant cette réaction chez certains sujets ou pouvant même être à l'origine de réactions phototoxiques ou photoallergiques.

Au cours de ses recherches, la demanderesse vient de découvrir que les dérivés hydrophiles du benzylidène camphre ayant la formule suivante :

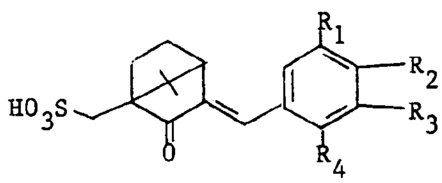

(I)

dans laquelle :

$R_1$ et $R_3$, identiques ou différents, représentent un atome d'hydrogène, un reste hydroxyle, un reste alkyle linéaire ou ramifié en $C_1$-$C_8$ ou un reste alcoxy linéaire ou ramifié en $C_1$-$C_8$, étant entendu que l'un au moins des radicaux $R_1$ et $R_3$ représente un reste hydroxyle, alkyle ou alcoxy;

$R_2$ et $R_4$, identiques ou différents, représentent un atome d'hydrogène ou un reste hydroxyle, étant entendu que l'un au moins des radicaux $R_2$ et $R_4$ représente un radical hydroxyle, sous forme d'acides libres ou de sels de métaux alcalins ou d'amines,

présentaient, de manière inattendue, outre de bonnes propriétés filtrantes dans la gamme de longueurs d'onde allant de 280 à 380 nm, d'excellentes propriétés anti-oxydantes vis-à-vis de la peroxydation des lipides polyinsaturés et également vis-à-vis des substances susceptibles de subir des réactions d'oxydation thermo- ou photoinduites (telles que des protéines, des sucres, des pigments, des vitamines, des polymères...).

Or, on sait que la peroxydation des lipides implique la formation de radicaux libres intermédiaires qui endommagent les membranes cellulaires se composant, entre autres, de phospholipides et sont responsables notamment des phénomènes de vieillissement de la peau (A.L TAPPEL dans "Federation Proceedings" Vol. 32, No 8, Août 1973).

Il est donc extrêmement intéressant de disposer de composés présentant à la fois des propriétés filtrantes dans une large bande et des propriétés anti-oxydantes potentialisant l'effet filtre. De tels composés peuvent permettre par exemple de mieux lutter contre le vieillissement prématuré de la peau dû à la peroxydation des lipides cutanés.

De tels composés peuvent aussi permettre d'assurer une meilleure conservation des compositions cosmétiques ou pharmaceutiques se présentant sous forme d'émulsions ou de lotions oléoalcooliques qui

comportent une phase grasse, en évitant le rancissement des lipides insaturés qui y sont contenus et qui peuvent être d'origine animale comme la lanoline, la cétine (blanc de baleine), la cire d'abeille, le perhydrosqualène, l'huile de tortue, ou végétale comme l'huile d'olive, l'huile de ricin, l'huile de maïs, l'huile d'amande douce, l'huile d'avocat, l'huile de karité, l'huile de tournesol, l'huile de soja, l'huile d'arachide, les huiles de coprah ou de palmiste, des acides gras essentiels comme la vitamine F et certaines huiles essentielles présentes dans les parfums comme l'huile de citron ou de lavande.

Ils peuvent également permettre d'éviter la dégradation oxydative de composés actifs contenus dans les compositions pharmaceutiques (vitamine A, caroténoïdes...)

La demanderesse a également découvert de manière surprenante, que les composés de formule (I) ci-dessus pouvaient être utilisés pour le traitement des inflammations et allergies cutanées.

Outre leurs bonnes propriétés filtrantes et anti-oxydantes, les composés (I) présentent un excellent caractère hydrophile ainsi qu'une très bonne stabilité thermique et photochimique.

Ces composés présentent également l'avantage de ne pas être toxiques ou irritants et d'avoir une parfaite innocuité vis-à-vis de la peau.

Certains des composés de formule (I) ci-dessus sont nouveaux.

Il s'agit des dérivés hydrophiles du benzylidène camphre de formule (I') suivante :

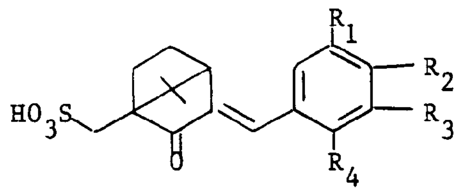

(I')

dans laquelle $R_1$ à $R_4$ ont les significations indiquées pour la formule (I), sous réserve que lorsque $R_1$ et $R_4$ représentent un atome d'hydrogène et $R_2$ un radical hydroxyle, $R_3$ ne représente pas un radical alcoxy, sous forme d'acides libres ou de sels alcalins ou d'amines.

Dans la formule (I'), $R_1$ et $R_3$ peuvent désigner par exemple un radical méthyle, éthyle, n-propyle, isopropyle, n-butyle, isobutyle, tertiobutyle ou 1,1,3,3-tétraméthylbutyle ou bien un reste méthoxy, éthoxy, propoxy, butoxy, pentyloxy, hexyloxy, heptyloxy ou octyloxy.

Parmi les composés préférés de formule (I'), on peut citer :

- l'acide 3',5'-ditert.-butyl-4'-hydroxy 3-benzylidène-campho-10-sulfonique,
- l'acide 3'-tert.-butyl-2'-hydroxy-5'-méthoxy-3-benzylidène-campho-10-sulfonique,
- l'acide 3'- tert.-butyl-2'-hydroxy-5'-méthyl-3-benzylidène-campho-10-sulfonique,

sous forme d'acides libres ou de sels alcalins ou d'amines.

La présente invention a donc pour objet les composés de formule (I') ci-dessus, sous forme d'acides libres ou de sels alcalins ou d'amines.

GB-A- 2 025 957 décrit, à titre seulement de titres solaires, certains dérivés de benzylidène camphre.

Les composés de formule (I) ou (I') sont obtenus à partir de l'acide campho-10-sulfonique synthétique (acide d,1-campho-10-sulfonique) ou à partir de l'acide campho-10-sulfonique naturel (acide d-campho-10-sulfonique) par condensation avec un aldéhyde aromatique de formule (II) selon le schéma réactionnel suivant :

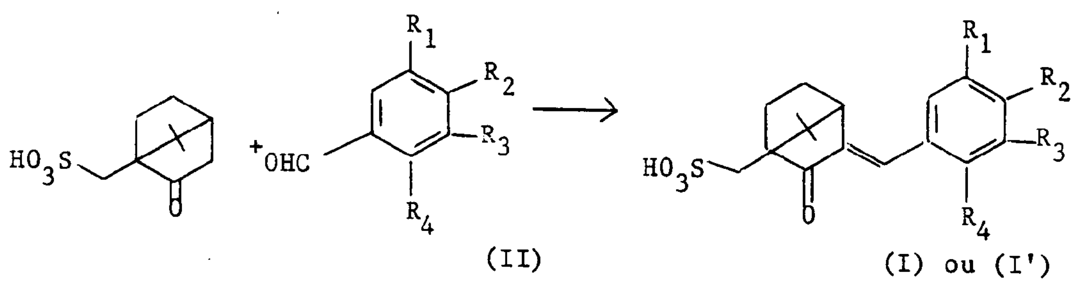

(II) (I) ou (I')

Les aldéhydes de formule (II) dans lesquels les substituants $R_1$ à $R_4$ ont les significations mentionnées ci-dessus pour les composés de formule (I) ou (I') sont des composés connus.

La condensation de l'aldéhyde (II) sur l'acide campho-10-sulfonique peut être effectuée en présence d'un alcoolate de métal alcalin tel que le méthylate de sodium ou le tertiobutylate de potassium, dans un solvant tel que le toluène, le 1,2-diméthoxyéthane ou le 1,2-diéthoxyéthane à une température comprise entre -78°C

et le point d'ébullition du solvant. La condensation peut également être effectuée en présence d'une base minérale telle qu'un amidure ou un hydrure de métal alcalin, en présence d'un solvant tel que le 1,2-diméthoxyéthane, ou un hydroxyde de métal alcalin dans le toluène ou un alcool, à une température comprise entre 0°C et le point d'ébullition du mélange réactionnel.

Un autre objet de l'invention est une composition cosmétique comprenant, dans un support cosmétiquement acceptable, une quantité efficace d'au moins un dérivé de benzylidène camphre de formule (I') ci-dessus, à titre d'agent anti-oxydant et filtrant les rayons UV de longueurs d'onde comprises entre 280 et 380 nm.

La composition cosmétique de l'invention peut être utilisée comme composition protectrice de l'épiderme humain ou des cheveux ou comme composition anti-solaire.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (I') contenu dans un support cosmétiquement acceptable.

On entend par "cheveux sensibilisés" des cheveux ayant subi un traitement de permanente, de coloration ou de décoloration.

L'invention a également pour objet une composition cosmétique colorée ou non colorée, stabilisée à la lumière et à l'oxydation, comprenant une quantité efficace d'au moins un dérivé de benzylidène camphre de formule (I') ci-dessus.

Lorsqu'elle est utilisée comme composition destinée à protéger l'épiderme humain contre les rayons ultraviolets ou comme composition antisolaire, la composition cosmétique selon l'invention peut se présenter sous les formes les plus diverses habituellement utilisées pour ce type de composition. Elle peut notamment se présenter sous forme de lotions aqueuses, hydroalcooliques ou oléoalcooliques, d'émulsions telles qu'une crème ou un lait, de gels aqueux, hydroalcooliques ou oléoalcooliques ou être conditionnée en aérosol pour former une mousse ou un spray.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (I') est présent dans des proportions en poids comprises entre 0,1 et 4% par rapport au poids total de la composition cosmétique protectrice de l'épiderme humain.

Comme solvant de solubilisation, on peut utiliser outre l'eau, un monoalcool ou un polyol inférieur ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (I'), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions aqueuses, hydroalcooliques ou oléoalcooliques; les lotions hydroalcooliques ou oléoalcooliques contiennent un alcool inférieur tel que l'éthanol ou un glycol tel que le propylèneglycol et/ou un polyol tel que la glycérine et de l'eau ou bien des huiles, cires et esters d'acides gras tels que les triglycérides d'acides gras.

La composition cosmétique de l'invention peut également être un gel aqueux, hydroalcoolique ou oléoalcoolique pouvant comprendre de l'eau, un ou plusieurs alcools ou polyols inférieurs tels que l'éthanol, le propylèneglycol ou la glycérine et un épaississant. Les gels oléoalcooliques contiennent en outre une huile ou une cire naturelle ou synthétique.

La présente invention vise également les compositions cosmétiques anti-solaires contenant au moins un composé de formule (I') et pouvant contenir d'autres filtres UV-B et/ou UV-A.

Dans ce cas, la quantité de filtre de formule (I') est comprise entre 0,2 et 15% en poids, la quantité totale de filtres présents dans la composition anti-solaire, c'est-à-dire le composé de formule (I') et éventuellement les autres filtres, étant comprise entre 0,5 et 15% en poids par rapport au poids total de la composition anti-solaire.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de laque pour cheveux, de composition de permanente, de coloration ou décoloration des cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousses, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 4% en poids de composé de formule (I′).

La présente invention vise également les compositions cosmétiques renfermant un constituant sensible à la lumière et/ou à l'oxydation et contenant au moins un composé de formule (I′) à titre d'agent de protection contre les rayons ultraviolets et agent anti-oxydant. Ces compositions sont constituées par des compositions capillaires telles que les laques pour cheveux, les lotions de mise en plis éventuellement traitantes ou démêlantes, les shampooings, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière et/ou à l'oxydation au cours du stockage.

De telles compositions contiennent 0,1 à 4% en poids de composé de formule (I′).

L'invention vise également un procédé de protection des compositions cosmétiques ou pharmaceutiques contre les rayons ultraviolets et l'oxydation, consistant à incorporer à ces compositions une quantité efficace d'au moins un composé de formule (I′).

Un autre objet de l'invention est l'utilisation des composés de formule (I′) en tant que filtres solaires à large bande absorbant dans la gamme de longueurs d'onde allant de 280 à 380 nm.

L'invention a également pour objet l'application à titre de produits cosmétiques des composés de formule (I′).

Un autre objet de l'invention est l'utilisation en tant qu'agents anti-oxydants des composés de formule (I).

L'effet anti-oxydant de ces composés peut être mis en évidence par chimiluminescence d'homogénats cellulaires. Cette technique est basée sur l'émission spontanée de lumière qui résulte de la désactivation radiative des produits de décomposition des lipides peroxydés.

L'étude cinétique s'effectue sur des broyats de cervelles de rats, dilués dans un tampon phosphate et est menée parallèlement avec une solution témoin ne contenant pas d'anti-oxydant et des solutions contenant l'anti-oxydant à différentes concentrations variant de $10^{-7}$ à $10^{-5}$ molaire.

Par référence au témoin, on peut déterminer les concentrations $C_x$ en anti-oxydant permettant l'inhibition de x% de la peroxydation.

Comme on l'a indiqué ci-dessus, la demanderesse a en outre découvert au cours de ses recherches, que les composés de formule (I) présentaient une activité pharmacologique intéressante dans le domaine du traitement des inflammations et allergies cutanées.

L'invention a donc pour objet le composé de formule (I) pour son utilisation comme médicament.

L'invention a également pour objet une composition pharmaceutique contenant une quantité efficace d'au moins un composé de formule (I) à titre d'ingrédient actif, dans un support ou un excipient non toxique.

A titre de composés (1) préférés, on peut citer :

- l'acide 3′,5′-ditert.-butyl-4′-hydroxy-3-benzylidène-campho-10-sulfonique,
- l'acide 3′-tert.-butyl-2′-hydroxy-5′-méthoxy-3-benzylidène-campho-10-sulfonique,
- l'acide 3′-tert.-butyl-2′-hydroxy-5′-méthyl-3-benzylidène-campho-10-sulfonique,

sous forme d'acides libres ou de sels alcalins ou d'amines.

La composition pharmaceutique conforme à l'invention peut être administrée par voie orale ou par voie topique.

Pour la voie orale, la composition pharmaceutique peut se présenter sous forme de comprimés, de gélules, de dragées, de sirops, de suspensions, de solutions, d'émulsions etc.. Pour la voie topique, la composition pharmaceutique selon l'invention se présente sous forme d'onguent, de crème, de pommade, de solution, de lotion, de gel, spray, suspension, etc.

Cette composition médicamenteuse peut contenir des additifs inertes ou pharmacodynamiquement actifs et notamment : des agents hydratants, des antibiotiques, des agents anti-inflammatoires stéroïdiens ou non stéroïdiens, des caroténoïdes, des agents anti-psoriasiques.

Cette composition peut également contenir des agents d'amélioration de la saveur, des agents conservateurs, des agents stabilisants, des agents régulateurs d'humidité, des agents régulateurs de pH, des agents modificateurs de la pression osmotique, des agents émulsionnants, des anesthésiques locaux, des tampons etc.

Elle peut aussi être conditionnée sous des formes retard ou à libération progressive connues en elles-mêmes.

Le composé de formule (I) conforme à l'invention est présent dans les compositions pharmaceutiques

dans des proportions comprises entre 0,01 et 80 % en poids, par rapport au poids total de la composition et de préférence entre 0,1 et 20 % en poids.

Dans l'application thérapeutique, le traitement est déterminé par le médecin et peut varier selon l'âge, le poids et la réponse du patient ainsi que la gravité des symptômes.

Lorsque les composés de formule (I) sont administrés par voie orale, le dosage est généralement compris entre 0,1 et 50 mg/kg/jour et de préférence entre 0,2 et 20 mg/kg/jour. La durée du traitement est variable suivant la gravité des symptômes et peut s'étaler entre 1 et 25 semaines, de façon continue ou discontinue.

Les compositions par voie topique contiennent de préférence de 0,25 % à 4 % en poids de composé de formule (I).

Comme support ou excipient de la composition pharmaceutique de l'invention, on peut utiliser tous supports ou excipients conventionnels non toxiques.

Les exemples suivants sont destinés à illustrer l'invention sans pour autant présenter un caractère limitatif.

EXEMPLES DE PREPARATION

EXEMPLE 1

Préparation de l'acide 3',5'-ditert.-butyl-4'-hydroxy-3-benzylidène-campho-10-sulfonique de formule

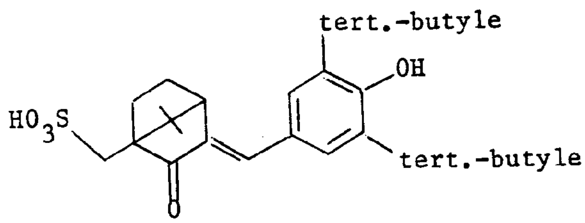

On chauffe au reflux pendant 15 minutes une suspension de 3,4 g (0,015 mole) d'acide d,1-campho-10-sulfonique et 5 g (0,045 mole) de tert.-butylate de potassium dans 50 cm³ de 1,2-diéthoxyéthane. On ajoute, sous agitation, 3,5 g (0,015 mole) de 3,5-ditert.-butyl-4-hydroxy benzaldéhyde et on maintient au reflux pendant 8 heures. Le mélange réactionnel est versé dans une solution aqueuse saturée de chlorure de sodium contenant 2 équivalents d'acide chlorhydrique. On extrait au dichlorométhane. Après évaporation du solvant, le résidu est agité pendant 1 heure, au bain marie bouillant, dans une solution d'acide chlorhydrique 6 N. Le précipité est filtré puis redissous dans l'eau chaude. La solution aqueuse est filtrée à chaud. Par concentration de la phase aqueuse, puis traitement par de l'acide chlorhydrique 12 N, on obtient un précipité gommeux qui est purifié, après séchage, par lavage à l'éther isopropylique. Le produit obtenu possède les caractéristiques suivantes :

- Analyse élémentaire : $C_{25}H_{36}O_5S$, $H_2O$

| | C% | H% | O% | S% |
|---|---|---|---|---|
| Calculé | 64,37 | 8,15 | 20,60 | 6,87 |
| Trouvé | 64,18 | 8,35 | 20,82 | 6,64 |

- Spectre UV (Eau)
  . $\lambda_{max}$ : 325 nm
  . $\varepsilon_{max}$ : 19900

EXEMPLE 2

Préparation de l'acide 3'-tert.-butyl-2'-hydroxy-5'-méthoxy-3-benzylidène-campho-10-sulfonique

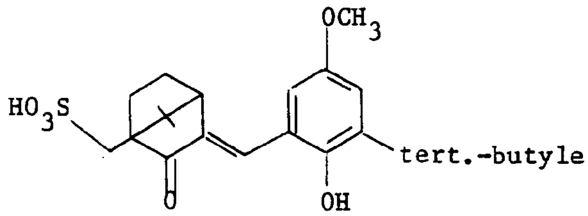

On chauffe au reflux pendant 1 heure 8,8 g (0,038 mole) d'acide d,1-campho-10-sulfonique, 6,1 g (0,114 mole) de méthylate de sodium et 7,9 g (0,038 mole) de 3-tert.-butyl-2-hydroxy-5-méthoxy benzaldéhyde dans 130 cm³ de 1,2-diméthoxyéthane. Après refroidissement, on ajoute, sous agitation, 2 équivalents d'acide chlorhydrique 2N. La phase aqueuse est saturée par du chlorure de sodium. On extrait au dichlorométhane, puis le solvant est évaporé. Le solide jaune ainsi obtenu est dispersé à chaud dans l'éther diisopropylique. On filtre puis sèche sous vide. Le sel de sodium ainsi obtenu est redissous dans l'eau et la solution est filtrée sur colonne de résine échangeuse d'ions Dowex 50. On obtient, après évaporation de l'eau sous pression réduite, à température ambiante, le composé attendu sous forme d'un solide orangé possédant les caractéristiques suivantes :

- Analyse élémentaire : $C_{22}H_{30}O_6S$

| | C % | H % | O% | S% |
|---|---|---|---|---|
| Calculé | 62,54 | 7,16 | 22,72 | 7,59 |
| Trouvé | 62,57 | 7,49 | 22,97 | 6,93 |

- Spectre UV (sous forme de sel de sodium, dans l'eau)
  - . $\lambda_{max1}$ : 295 nm
  - . $\varepsilon_{max1}$ : 11150
  - . $\lambda_{max2}$ : 355 nm
  - . $\varepsilon_{max2}$ : 7500

EXEMPLE 3

Préparation de l'acide 3'-tert.-butyl-2'-hydroxy-5'-méthyl-3-benzylidène-campho-10-sulfonique de formule :

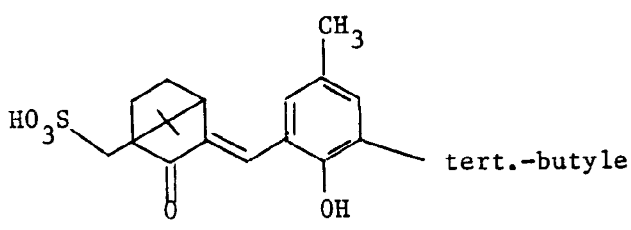

On chauffe au reflux pendant 15 minutes une suspension de 3,4 g (0,015 mole) d'acide d,1-campho-10-sulfonique et 5 g (0,045 mole) de tert.-butylate de potassium dans 50 cm³ de 1,2-diéthoxyéthane. On ajoute, sous agitation, 3,1 g (0,015 mole) de 3-tert.-butyl-2-hydroxy-5-méthyl benzaldéhyde. On porte au reflux pendant 30 minutes en distillant environ 30 cm³ de 1,2-diéthoxyéthane puis on maintient au reflux pendant 2 heures 30 minutes. Le mélange réactionnel est versé dans une solution aqueuse saturée de chlorure de sodium contenant 2 équivalents d'acide chlorhydrique. On extrait au toluène. Après évaporation du solvant, le résidu est mis en suspension dans 250 cm³ d'acide chlorhydrique 6N. On chauffe au bain-marie bouillant jusqu'à solidification. Après filtration, lavage à l'eau puis à l'éther et séchage, on obtient 1,4 g de produit jaune vif possédant les caractéristiques suivantes :

- Analyse élémentaire : $C_{22}H_{30}O_5S$

| | C% | H% | O% | S% |
|---|---|---|---|---|
| Calculé | 65,00 | 7,44 | 19,68 | 7,89 |
| Trouvé | 64,92 | 7,67 | 19,78 | 7,77 |

Spectre UV (eau)
. $\lambda_{max}$ : 295 nm
. $\varepsilon_{max}$ : 12640

Mise en évidence des propriétés anti-oxydantes par chimiluminescence d'homogénats cellulaires

Le test de chimiluminescence est exécuté selon la publication de E.A. LISSI, T. CACERES et L.A. VIDELA "Visible Chemiluminescence from rat brain homogenates undergoing autoxidation. I. Effect of additives and products accumulation" dans "Journal of Free Radicals in Biology and Medicine", Vol.2, pp. 63-69 (1986). Ce test a été simplifié dans le sens qu'on a mesuré uniquement la chimiluminescence sans mesurer l'accumulation de l'acide thiobarbiturique après 1 heure d'irradiation. Cependant, selon la publication, il y a une bonne corrélation entre la chimiluminescence et la mesure de l'accumulation de l'acide thiobarbiturique.

Des cervelles de rats sont prélevées immédiatement après dislocation des cervicales. Elles sont lavées avec une solution tampon contenant 140 mM de NaCl et 40mM de phosphate de potassium, débarrassées des vaisseaux apparents et de la membrane extérieure , puis broyées avec 4 fois leur volume de solution tampon. Ce broyat est dilué 3 fois avec la solution tampon (dilution totale 12 fois par rapport aux organes de départ).

10 ml d'homogénat dilué est introduit dans des flacons de comptage contenant 200 µl de solution du produit anti-oxydant à tester dissous à différentes concentrations dans du méthanol ou du diméthylsulfoxyde. Immédiatement après agitation, les flacons sont introduits dans un compteur de scintillation (BECKMAN) et comptés périodiquement.

Parallèlement, une étude est menée sur une solution témoin ne contenant pas d'anti-oxydant.

On établit ainsi, pour chaque concentration, une courbe d'intensité de chimiluminescence en fonction du temps.

Par comparaison des pentes des courbes obtenues avec les solutions à différentes concentrations et la solution témoin, on détermine $C_{50}$, c'est-à-dire la concentration en anti-oxydant permettant l'inhibition de 50% de la peroxydation.

Composé de l'exemple 1 : $C_{50}$ = 1,50.$10^{-6}$M

Composé de l'exemple 2 : $C_{50}$ = 0,70.$10^{-6}$M
(sous forme de sel de sodium).

Testé dans les mêmes conditions, le ditert.-butyl-hydroxy toluène (BHT), qui est un anti-oxydant classique, a une $C_{50}$ de 3,20.$10^{-6}$M.

EXEMPLES D'APPLICATION

EXEMPLE A - Lait antisolaire (émulsion eau-dans-l'huile)

| | | |
|---|---|---|
| - Composé de l'exemple 1 | | 0,5 g |
| - Benzylidène camphre | | 2,0 g |
| - Mélange d'esters d'acides gras, d'esters polyglycérolés et de tensio-actifs siliconés "ABIL WS08" (GOLDSCHMIDT) | | 5,0 g |
| - Vaseline blanche | | 5,0 g |
| - Cire d'abeilles | | 5,0 g |
| - Octyldodécanol "EUTANOL G" (HENKEL) | | 7,0 g |
| - Huile de vaseline | | 19,0 g |
| - Glycérine | | 5,0 g |
| - Parfum | | 0,4 g |
| - Conservateur | | 0,2 g |
| - Eau déminéralisée | qsp | 100 g |

On dissout le composé de l'exemple 1 et le benzylidène camphre dans les corps gras et l'émulsionnant et on chauffe à 70-80°C; on chauffe à la même température la phase aqueuse constituée par l'eau, le chlorure de sodium et la glycérine; on ajoute sous vive agitation, la phase aqueuse à la phase grasse; puis on laisse refroidir sous agitation modérée et vers 40°C, on ajoute parfum et conservateur.

EXEMPLE B - Lait anti-solaire (émulsion huile-dans-l'eau)

| | | |
|---|---|---|
| - Composé de l'exemple 1 | | 1,5 g |
| - p-méthoxycinnamate de 2-éthylhexyle "PARSOL MCX" | | 3,5 g |
| - 2-hydroxy-4-méthoxy-benzophénone "UVINUL M 40" | | 1,0 g |
| - Alcool cétylique | | 1,0 g |
| - Alcool oléocétylique à 30 moles OE : "MERGITAL OC 30" (HENKEL) | | 5,0 g |
| - Alcool stéarylique | | 4,0 g |
| - Huile de synthèse de formule : $C_{15}H_{31}COO\ CH_2-CH(OH)-CH_2-O-CH_2-CH(C_2H_5)-C_4H_9$ | | 2,0 g |
| - Mélange 90/10 de 2-éthyl-hexanoate de cétostéaryle et de myristate d'isopropyle "CERAMOLL" (CREATIONS AROMATIQUES) | | 2,0 g |
| - Huile de vaseline | | 8,0 g |
| - Propylène glycol | | 4,0 g |
| - Conservateur | | 0,2 g |
| - Parfum | | 0,4 g |
| - Eau déminéralisée | qsp | 100 g |

On dissout le composé de l'exemple 1 et les filtres dans les corps gras vers 70-80°C; on chauffe à la même température la phase aqueuse constituée par l'eau, le propylène glycol et l'émulsionnant et on ajoute sous vive agitation la phase grasse à la phase aqueuse, puis on laisse refroidir sous agitation modérée et vers 40°C, on ajoute le conservateur et le parfum.

EXEMPLE C - Gel protecteur de la peau

| | | |
|---|---|---|
| - Composé de l'exemple 1 | | 0,1 g |
| - Acide polyacrylique réticulé vendu sous la dénomination "CARBOPOL 934" (GOODRICH CHEMICAL) | | 0,8 g (MA) |
| - Glycérol | | 12,0 g |
| - Alcool éthylique absolu | | 15 g |
| - Conservateur | | 0,2 g |
| - Parfum | | 0,2 g |
| - Soude à 20% | qs | pH = 5,3 |
| - Eau | qsp | 100 g |

EXEMPLE D - Crème anti-inflammatoire

| | | |
|---|---|---|
| - Composé de l'exemple 1 neutralisé par la triéthanolamine | | 2,0 g |
| - Dodécylsulfate de sodium | | 0,8 g |
| - Glycérol | | 2,0 g |
| - Alcool stéarylique | | 20,0 g |
| - Triglycérides d'acides caprique/caprylique vendus par la Société DYNAMIT NOBEL sous la dénomination "MIGLYOL 812" | | 20,0 g |
| - Conservateur | qs | |
| - Eau déminéralisée stérile | qsp | 100 g |

La phase aqueuse contenant les composés hydrosolubles et le composé de l'exemple 1 neutralisé est chauffée vers 80°C; la phase grasse est également chauffée à 80°C.

Sous vive agitation, on ajoute la phase grasse à la phase aqueuse et on laisse refroidir sous agitation modérée.

## Revendications

**1.** Dérivé de benzylidène camphre caractérisé par le fait qu'il répond à la formule :

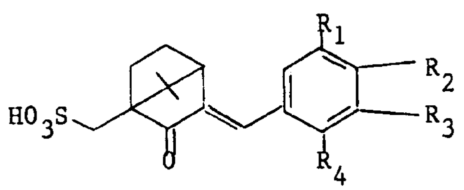

(I')

dans laquelle $R_1$ et $R_3$, identiques ou différents, représentent un atome d'hydrogène, un radical hydroxyle, un radical alkyle linéaire ou ramifié en $C_1$-$C_8$ ou un radical alcoxy en $C_1$-$C_8$ linéaire ou ramifié, étant entendu que l'un au moins des radicaux $R_1$ et $R_3$ représente un radical hydroxyle, alkyle ou alcoxy;

$R_2$ et $R_4$, identiques ou différents, représentent un atome d'hydrogène ou un radical hydroxyle, étant entendu que l'un au moins des symboles $R_2$ et $R_4$ représente un radical hydroxyle,

sous réserve que lorsque $R_1$ et $R_4$ représentent un atome d'hydrogène et $R_2$ un radical hydroxyle, $R_3$ ne représente pas un radical alcoxy,

ainsi que ses sels alcalins ou d'amines.

**2.** Composé selon la revendication 1, caractérisé par le fait qu'il est choisi parmi l'acide 3′,5′-ditert.-butyl-4′-hydroxy-3-benzylidène-campho-10-sulfonique, l'acide 3′-tert.-butyl-2′-hydroxy-5′-méthoxy-3-benzylidène-campho-10-sulfonique, l'acide 3′-tert.-butyl-2′-hydroxy-5′-méthyl-3-benzylidène-campho-10-sulfonique et leurs sels alcalins ou d'amines.

**3.** Composition cosmétique, caractérisée par le fait qu'elle comprend, dans un support cosmétiquement acceptable, une quantité efficace d'au moins un composé de formule (I′) selon la revendication 1 ou 2, à titre d'agent anti-oxydant et filtrant les rayons UV de longueurs d'onde comprises entre 280 et 380 nm..

**4.** Composition cosmétique selon la revendication 3, caractérisée par le fait qu'elle comprend à titre de composé (I′), au moins l'un des composés choisis parmi : l'acide 3′-5′-ditert.-butyl-4′-hydroxy-3-benzylidène-campho-10-sulfonique, l'acide 3′-tert.-butyl-2′-hydroxy-5′-méthoxy-3-benzylidène-campho-10-sulfonique, l'acide 3′-tert.-butyl-2′-hydroxy-5′-méthyl-3-benzylidènecampho-10-sulfonique ainsi que leurs

sels alcalins ou d'amines.

**5.** Composition cosmétique selon la revendication 3 ou 4, caractérisée par le fait qu'elle se présente sous forme de lotion aqueuse, hydroalcoolique ou oléoalcoolique, d'émulsion, de gel aqueux, oléoalcoolique ou hydroalcoolique, ou d'aérosol.

**6.** Composition cosmétique selon la revendication 5, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, propulseurs, colorants et pigments.

**7.** Composition cosmétique selon la revendication 5 ou 6, caractérisée par le fait qu'elle constitue une composition protectrice de l'épiderme humain et contient 0,1 à 4% en poids de composé de formule (I').

**8.** Composition cosmétique selon la revendication 5 ou 6, se présentant sous forme de composition antisolaire, caractérisée par le fait qu'elle contient 0,2 à 15% en poids de composé de formule (I').

**9.** Composition cosmétique anti-solaire selon la revendication 8, caractérisée par le fait qu'elle contient en outre un agent filtrant les rayons UV-B et/ou UV-A.

**10.** Composition cosmétique selon la revendication 3 ou 4, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou de coloration et comprend 0,25 à 4% en poids de composé de formule (I').

**11.** Composition cosmétique selon la revendication 3 ou 4, se présentant sous forme d'une composition cosmétique colorée ou non, stabilisée à la lumière et à l'oxydation, caractérisée par le fait qu'elle est constituée par une composition capillaire, un produit de maquillage ou une composition pour les soins ou le traitement de la peau, comprenant 0,1 à 4% en poids de composé de formule (I').

**12.** Utilisation d'une composition cosmétique contenant au moins un dérivé de benzylidène camphre de formule (I') de la revendication 1 ou 2 pour la protection de la peau et des cheveux naturels ou sensibilisés contre le rayonnement ultraviolet, par l'application sur la peau ou les cheveux d'une quantité efficace de ladite composition.

**13.** Procédé de protection d'une composition cosmétique ou pharmaceutique contre les rayons ultra-violets et l'oxydation, caractérisé par le fait qu'il consiste à incorporer à cette composition une quantité efficace d'au moins un composé de formule (I').

**14.** Utilisation d'au moins un composé de formule (I') selon la revendication 1 ou 2 à titre de produit cosmétique.

**15.** Utilisation d'au moins un composé de formule (I') selon la revendication 1 ou 2 en tant que filtre solaire à large bande absorbant dans la gamme de longueurs d'onde allant de 280 à 380 nm.

**16.** Utilisation en tant qu'agent anti-oxydant d'au moins un composé de formule (I) suivante :

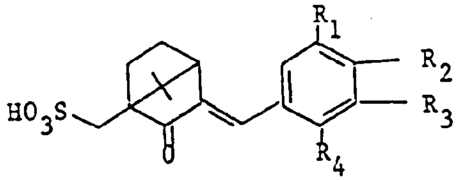

(I)

dans laquelle $R_1$ et $R_3$, identiques ou différents, représentent un atome d'hydrogène, un reste hydroxyle, un reste alkyle linéaire ou ramifié en $C_1$-$C_8$ ou un reste alcoxy linéaire ou ramifié en $C_1$-$C_8$, étant entendu que l'un au moins des radicaux $R_1$ et $R_3$ représente un reste hydroxyle, alkyle ou alcoxy;

$R_2$ et $R_4$, identiques ou différents, représentent un atome d'hydrogène ou un reste hydroxyle, étant entendu que l'un au moins des radicaux $R_2$ et $R_4$ représente un radical hydroxyle,

sous forme d'acide libre ou de sel de métaux alcalins ou d'amines.

**17.** Utilisation en tant qu'agent anti-oxydant d'au moins un composé de formule (I) choisi parmi l'acide 3',5'-ditert.-butyl-4'-hydroxy-3-benzylidène-campho-10-sulfonique, l'acide 3'-tert.-butyl-2'-hydroxy-5'-méthoxy-3-benzylidène-campho-10-sulfonique, l'acide 3'-tert.-butyl-2'-hydroxy-5'-méthyl-3-benzylidène-campho-10-sulfonique et leurs sels alcalins ou d'amines.

**18.** Composé de formule (I) :

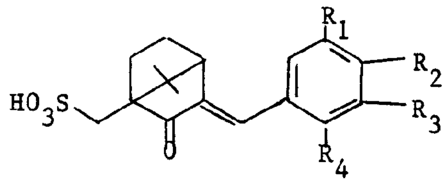

(I)

dans laquelle

$R_1$ et $R_3$, identiques ou différents, représentent un atome d'hydrogène, un radical hydroxyle, un radical alkyle linéaire ou ramifié en $C_1$-$C_8$ ou un radical alcoxy linéaire ou ramifié en $C_1$-$C_8$, étant entendu que l'un au moins des radicaux $R_1$ et $R_3$ représente un radical hydroxyle, alkyle ou alcoxy,

$R_2$ et $R_4$, identiques ou différents, représentent un atome d'hydrogène ou un radical hydroxyle, étant entendu que l'un au moins des radicaux $R_2$ et $R_4$ représente un radical hydroxyle,

pour son utilisation comme médicament.

**19.** Composé de formule (I) selon la revendication 18, caractérisé par le fait qu'il est choisi parmi l'acide 3',5'-ditert.-butyl-4'-hydroxy-3-benzylidène-campho-10-sulfonique, l'acide 3'-tert.-butyl-2'-hydroxy-5'-méthoxy-3-benzylidène-campho-10-sulfonique, l'acide 3'-tert.-butyl-2'-hydroxy-5'-méthyl-3-benzylidène-campho-10-sulfonique et leurs sels alcalins ou d'amines.

**20.** Composé de formule (I) selon la revendication 18 ou 19 pour son utilisation dans le traitement des inflammations et allergies cutanées.

**21.** Composition pharmaceutique, caractérisée par le fait qu'elle comprend une quantité efficace d'au moins un composé de formule (I) selon la revendication 18 ou 19, dans un support ou excipient non toxique.

**22.** Composition pharmaceutique destinée à être administrée par voie topique, se présentant sous forme de crème, onguent, pommade, solution, gel, lotion, spray, suspension, caractérisée par le fait qu'elle contient au moins un composé de formule (I) selon la revendication 18 ou 19.

**23.** Composition pharmaceutique destinée à être administrée par voie orale sous forme de comprimés, de gélules, de dragées, de sirops, de suspensions, de solutions, d'émulsions, caractérisée par le fait qu'elle contient au moins un composé de formule (I) selon la revendication 18 ou 19.

**24.** Composition pharmaceutique selon la revendication 22, caractérisée par le fait qu'elle contient le composé actif de formule (I) dans des proportions comprises entre 0,25 et 4% en poids par rapport au poids total de la composition.

**25.** Utilisation d'un composé de formule (I) selon la revendication 18 pour la préparation d'un médicament destiné au traitement des inflammations et allergies cutanées.

## Patentansprüche

**1.** Benzylidenkampferderivat der Formel :

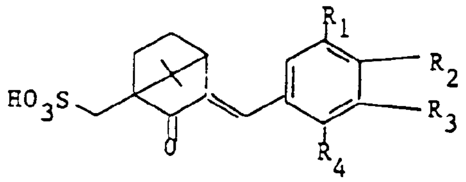

(I')

worin $R_1$ und $R_3$, gleich oder verschieden, ein Wasserstoffatom, einen Hydroxylrest, einen linearen oder verzweigten $C_{1-8}$-Alkylrest oder einen linearen oder verzweigten $C_{1-8}$-Alkoxyrest darstellen, wobei mindestens einer der Reste $R_1$ und $R_3$ einen Hydroxyl-, Alkyl- oder Alkoxyrest darstellt,
$R_2$ und $R_4$, gleich oder verschieden, ein Wasserstoffatom oder einen Hydroxylrest darstellen, wobei mindestens einer der Reste $R_2$ und $R_4$ einen Hydroxylrest darstellt,
mit der Maßgabe, daß, wenn $R_1$ und $R_4$ ein Wasserstoffatom und $R_2$ einen Hydroxylrest darstellen, $R_3$ keinen Alkoxyrest darstellt,
sowie dessen Alkali- und Aminsalze.

2. Verbindung gemäß Anspruch 1,
dadurch **gekennzeichnet**, daß
sie ausgewählt ist aus:
- 3′,5′-Di-t-butyl-4′-hydroxy-3-benzylidenkampho-10-sulfonsäure,
- 3′-t-Butyl-2′-hydroxy-5′-methoxy-3-benzylidenkampho-10-sulfonsäure,
- 3′-t-Butyl-2′-hydroxy-5′-methyl-3-benzylidenkampho-10-sulfonsäure,
sowie aus deren Alkali- und Aminsalzen.

3. Kosmetische Zusammensetzung,
dadurch **gekennzeichnet**, daß
sie, in einem kosmetisch verträglichen Trägermedium, eine wirksame Menge mindestens einer Verbindung der Formel (I′) gemäß Anspruch 1 oder 2 als Antioxidans enthält, welches UV-Strahlen von Wellenlängen von 280 bis 380 nm filtert.

4. Kosmetische Zusammensetzung gemäß Anspruch 3,
dadurch **gekennzeichnet**, daß
sie als Verbindung (I′) mindestens eine der Verbindungen enthält, die ausgewählt sind aus:
- 3′,5′-Di-t-butyl-4′-hydroxy-3-benzylidenkampho-10-sulfonsäure,
- 3′-t-Butyl-2′-hydroxy-5′-methoxy-3-benzylidenkampho-10-sulfonsäure,
- 3′-t-Butyl-2′-hydroxy-5′-methyl-3-benzylidenkampho-10-sulfonsäure,
sowie aus deren Alkali- oder Aminsalzen.

5. Kosmetische Zusammensetzung gemäß Anspruch 3 oder 4,
dadurch **gekennzeichnet**, daß
sie in Form einer wässrigen, hydroalkoholischen oder oleoalkoholischen Lotion, einer Emulsion, eines wässrigen, oleoalkoholische oder hydroalkoholischen Gels oder eines Aerosols vorliegt.

6. Kosmetische Zusammensetzung gemäß Anspruch 5,
dadurch **gekennzeichnet**, daß
sie außerdem kosmetische Hilfsstoffe enthält, ausgewählt aus Verdickungsmitteln, Weichmachern, Feuchtigkeitsmitteln, oberflächenaktiven Mitteln, Konservierungsstoffen, anti-Schaummitteln, Parfüm-Produkten, Ölen, Wachsen, Lanolin, Niedrigmonoalkoholen und -polyolen, Treibmitteln, Färbemitteln und Pigmenten.

7. Kosmetische Zusammensetzung gemäß Anspruch 5 oder 6,
dadurch **gekennzeichnet**, daß
sie eine Zusammensetzung zum Schutz der menschlichen Haut darstellt und 0,1 bis 4 Gew.% der Verbindung der Formel (I′) enthält.

8. Kosmetische Zusammensetzung gemäß Anspruch 5 oder 6,
welche in Form einer Sonnenschutzmittel-Zusammensetzung vorliegt,
dadurch **gekennzeichnet**, daß

sie 0,2 bis 15 Gew.% der Verbindung der Formel (I′) enthält.

9. Kosmetische Sonnenschutzmittel-Zusammensetzung gemäß Anspruch 8, dadurch **gekennzeichnet**, daß sie ausserdem ein Filtermittel für UV-B- und/oder UV-A-Strahlen enthält.

10. Kosmetische Zusammensetzung gemäß Anspruch 3 oder 4 zur Aufbringung auf die Haare, dadurch **gekennzeichnet**, daß sie in Form von Schampoo, Lotion, Gel oder Emulsion zur Spülung, Frisier- oder Behandlungs-Lotion oder -Gel, Lotion oder Gel zum Bürsten oder zur Wellengebung, Haarlack oder einer Zusammensetzung zur Dauerwelle, Entfärbung oder Färbung vorliegt und 0,25 bis 4 Gew.% der Verbindung der Formel (I′) enthält.

11. Kosmetische Zusammensetzung gemäß Anspruch 3 oder 4, welche in Form einer gefärbten oder nicht-gefärbten kosmetischen Zusammensetzung vorliegt, die gegenüber der Einwirkung von Licht und Oxidation stabilisiert ist, dadurch **gekennzeichnet**, daß sie durch eine Haarmittel-Zusammensetzung, ein Schminkprodukt oder eine Zusammensetzung zur Pflege oder Behandlung der Haut dargestellt ist, welche 0,1 bis 4 Gew.% der Verbindung der Formel (I′) enthält.

12. Verwendung einer kosmetischen Zusammensetzung, die mindestens ein Benzylidenkampferderivat der Formel (I′) des Anspruchs 1 oder 2 enthält, zum Schutz der Haut und natürlicher oder sensibilisierter Haare vor ultravioletter Strahlung, und zwar durch Aufbringung einer wirksamen Menge der genannten Zusammensetzung auf die Haut oder die Haare.

13. Verfahren zur Schutz einer kosmetischen oder pharmazeutischen Zusammensetzung vor ultravioletten Strahlen und Oxidation, dadurch **gekennzeichnet**, daß man in diese Zusammensetzung eine wirksame Menge mindestens einer Verbindung der Formel (I′) einbringt.

14. Verwendung mindestens einer Verbindung der Formel (I′) gemäß Anspruch 1 oder 2 als kosmetisches Produkt.

15. Verwendung mindestens einer Verbindung der Formel (I′) gemäß Anspruch 1 oder 2 als Sonnenfilterstoff mit großer Bande, absorbierend im Wellenlängenbereich von 280 bis 380 nm.

16. Verwendung als Antioxidans mindestens einer Verbindung der folgenden Formel (I):

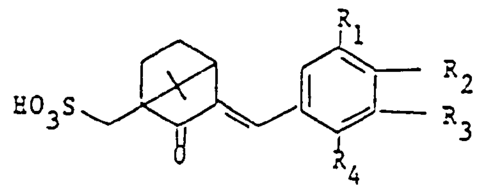

(I)

worin $R_1$ und $R_3$, gleich oder verschieden, ein Wasserstoffatom, einen Hydroxylrest, einen linearen oder verzweigten $C_{1-8}$-Alkylrest oder einen linearen oder verzweigten $C_{1-8}$-Alkoxyrest darstellen, wobei mindestens einer der Reste $R_1$ und $R_3$ einen Hydroxyl-, Alkyl- oder Alkoxyrest darstellt, $R_2$ und $R_4$, gleich oder verschieden, ein Wasserstoffatom oder einen Hydroxylrest darstellen, wobei mindestens einer der Reste $R_2$ und $R_4$ einen Hydroxylrest darstellt, in Form freier Säuren oder von Alkalimetall- oder Aminsalzen.

17. Verwendung als Antioxidans mindestens einer Verbindung der Formel (I), ausgewählt aus - 3′,5′-Di-t-butyl-4′-hydroxy-3-benzylidenkampho-10-sulfonsäure,
   - 3′-t-Butyl-2′-hydroxy-5′-methoxy-3-benzylidenkampho-10-sulfonsäure,
   - 3′-t-Butyl-2′-hydroxy-5′-methyl-3-benzylidenkampho-10-sulfonsäure,

sowie aus deren Alkali- oder Aminsalzen.

**18.** Verbindung der Formel (I) :

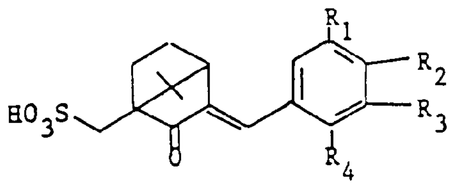

(I)

worin $R_1$ und $R_3$, gleich oder verschieden, ein Wasserstoffatom, einen Hydroxylrest, einen linearen oder verzweigten $C_{1-8}$-Alkylrest oder einen linearen oder verzweigten $C_{1-8}$-Alkoxyrest darstellen, wobei mindestens einer der Reste $R_1$ und $R_3$ einen Hydroxyl-, Alkyl- oder Alkoxyrest darstellt,
$R_2$ und $R_4$, gleich oder verschieden, ein Wasserstoffatom oder einen Hydroxylrest darstellen, wobei mindestens einer der Reste $R_2$ und $R_4$ einen Hydroxylrest darstellt,
zur Verwendung als Medikament.

**19.** Verbindung der Formel (I) gemäß Anspruch 18,
dadurch **gekennzeichnet**, daß
sie ausgewählt ist aus
- 3',5'-Di-t-butyl-4'-hydroxy-3-benzylidenkampho-10-sulfonsäure,
- 3'-t-Butyl-2'-hydroxy-5'-methoxy-3-benzylidenkampho-10-sulfonsäure,
- 3'-t-Butyl-2'-hydroxy-5'-methyl-3-benzylidenkampho-10-sulfonsäure,

sowie aus deren Alkali- oder Aminsalzen.

**20.** Verbindung der Formel (I) gemäß Anspruch 18 oder 19 zur Verwendung bei der Behandlung von Hautentzündungen und -allergien.

**21.** Pharmazeutische Zusammensetzug,
dadurch **gekennzeichnet**, daß
sie eine wirksame Menge mindestens einer Verbindung der Formel (I) gemäß Anspruch 18 oder 19, in einem nichttoxischen Trägermedium oder Expzipient, enthält.

**22.** Pharmazeutische Zusammensetzung zur topischen Verabreichung, die in Form einer Creme, Salbe, Pommade, Lösung, eines Gels, einer Lotion, eines Spray oder einer Suspension vorliegt,
dadurch **gekennzeichnet**, daß
sie mindestens eine Verbindung der Formel (I) gemäß Anspruch 18 oder 19 enthält.

**23.** Pharmazeutische Zusammensetzung zur oralen Verabreichung in Form von Tabletten, Kapseln, Dragees, Sirups, Suspensionen, Lösungen oder Emulsionen,
dadurch **gekennzeichnet**, daß
sie mindestens eine Verbindung der Formel (I) gemäß Anspruch 18 oder 19 enthält.

**24.** Pharmazeutische Zusammensetzung gemäß Anspruch 22,
dadurch **gekennzeichnet**, daß
sie die Wirkstoffverbindung der Formel (I) in Mengenverhältnissen von 0,25 bis 4 Gew.%, bezogen auf Gesamtgewicht der Zusammensetzung, enthält.

**25.** Verwendung einer Verbindung der Formel (I) gemäß Anspruch 18 zur Herstellung eines Medikaments zur Behandlung von Hautentzündungen und -allergien.

## Claims

**1.** Benzylidenecamphor derivative, characterized in that it corresponds to the formula:

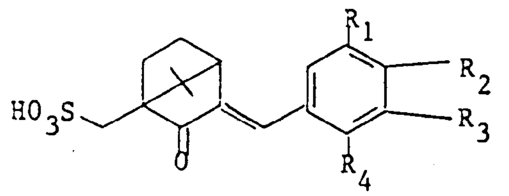

(I′)

in which $R_1$ and $R_3$, which may be identical or different, represent a hydrogen atom, a hydroxyl radical, a linear or branched $C_1$-$C_8$ alkyl radical or a linear or branched $C_1$-$C_8$ alkoxy radical, on the understanding that at least one of the radicals $R_1$ and $R_3$ represents a hydroxyl, alkyl or alkoxy radical; and

$R_2$ and $R_4$, which may be identical or different, represent a hydrogen atom or a hydroxyl radical, on the understanding that at least one of the symbols $R_2$ and $R_4$ represent a hydroxyl radical,

with the proviso that, when $R_1$ and $R_4$ represent a hydrogen atom and $R_2$ a hydroxyl radical, $R_3$ does not represent an alkoxy radical,

as well as its alkali metal or amine salts.

2. Compound according to Claim 1, characterized in that it is selected from 3′,5′-di-tert-butyl-4′-hydroxy-3-benzylidene-10-camphorsulphonic acid, 3′-tert-butyl-2′-hydroxy-5′-methoxy-3-benzylidene-10-camphorsulphonic acid, 3′-tert-butyl-2′-hydroxy-5′-methyl-3-benzylidene-10-camphorsulphonic acid and their alkali metal or amine salts.

3. Cosmetic composition, characterized in that it comprises, in a cosmetically acceptable vehicle, an effective amount of at least one compound of formula (I′) according to Claim 1 or 2, by way of an antioxidant agent and agent screening out UV rays of wavelengths between 280 and 380 nm.

4. Cosmetic composition according to Claim 3, characterized in that it comprises, by way of a compound (I′), at least one of the compounds selected from: 3′,5′-di- tert-butyl-4′-hydroxy-3-benzylidene-10-camphorsulphonic acid, 3′-tert-butyl-2′-hydroxy-5′-methoxy-3-benzylidene-10-camphorsulphonic acid, 3′-tert-butyl-2′-hydroxy-5′-methyl-3-benzylidene-10-camphorsulphonic acid and also their alkali metal or amine salts.

5. Cosmetic composition according to Claim 3 or 4, characterized in that it is presented in the form of an aqueous, aqueous-alcoholic or oleoalcoholic lotion, an emulsion, an aqueous, oleoalcoholic or aqueous-alcoholic gel or an aerosol.

6. Cosmetic composition according to Claim 5, characterized in that it contains, in addition, cosmetic adjuvants selected from thickeners, emollients, humectants, surfactants, preservatives, antifoams, fragrances, oils, waxes, lanolin, lower monohydric alcohols and polyols, propellants, colourings and pigments.

7. Cosmetic composition according to Claim 5 or 6, characterized in that it constitutes a composition for protecting the human epidermis and contains 0.1 to 4% by weight of compound of formula (I).

8. Cosmetic composition according to Claim 5 or 6, presented in the form of an antisun composition, characterized in that it contains 0.2 to 15% by weight of compound of formula (I′).

9. Antisun cosmetic composition according to Claim 8, characterized in that it contains, in addition, an agent screening out UV-B and/or UV-A rays.

10. Cosmetic composition according to Claim 3 or 4, intended for application on the hair, characterized in that it is presented in the form of a shampoo, lotion, gel or emulsion to be rinsed, styling or treatment lotion or gel, blow-drying or setting lotion or gel, hair lacquer or permanent-waving, bleaching or dyeing composition, and comprises 0.25 to 4% by weight of compound of formula (I′).

11. Cosmetic composition according to Claim 3 or 4, presented in the form of a coloured or uncoloured cosmetic composition, stabilized to light and to oxidation, characterized in that it consists of a hair-care composition, a makeup product or a skin-care or -treatment composition, comprising 0.1 to 4% by weight of compound of formula (I′).

12. Use of a cosmetic composition containing at least a benzylidenecamphor of formula (I') according to claim 1 or 2 for protecting the skin and natural or sensitized hair against ultraviolet radiation by application on the skin or on the hair of an effective amount of this composition.

13. Process for protecting a cosmetic or pharmaceutical composition against ultraviolet rays and oxidation, characterized in that it consists in incorporating an effective amount of at least one compound of formula (I') in this composition.

14. Use of at least one compound of formula (I') according to Claim 1 or 2 by way of a cosmetic product.

15. Use of at least one compound of formula (I') according to Claim 1 or 2 as a broad-band sunscreen absorbing in the wavelength range extending from 280 to 380 nm.

16. Use as an antioxidant agent of at least one compound of the following formula (I) :

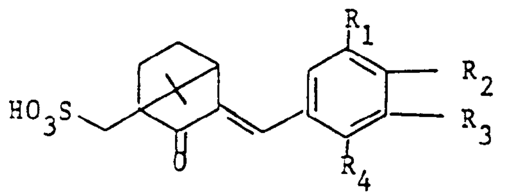

(I)

in which $R_1$ and $R_3$, which may be identical or different, represent a hydrogen atom, a hydroxyl residue, a linear or branched $C_1$-$C_8$ alkyl residue or a linear or branched $C_1$-$C_8$ alkoxy residue, on the understanding that a least one of the- radicals $R_1$ and $R_3$ represents a hydroxyl, alkyl or alkoxy residue and

$R_2$ and $R_4$, which may be identical or different, represent a hydrogen atom or a hydroxyl residue, on the understanding that at least one of the radicals $R_2$ and $R_4$ represent a hydroxyl radical,

in the form of the free acid or of an alkali metal or amine salt.

17. Use as an antioxidant agent of at least one compound of formula (I) selected from 3',5'-di-tert butyl-4'-hydroxy-3-benzylidene-10-camphorsulphonic acid, 3'-tert-butyl-2'-hydroxy-5'-methoxy-3-benzylidene-10-camphorsulphonic acid, 3'-tert-butyl-2'-hydroxy-5'-methyl-3-benzylidene-10-camphorsulphonic acid and their alkali metal or amine salts.

18. Compound of formula (I):

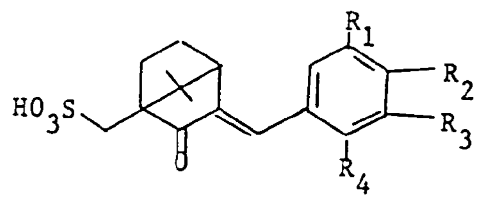

(I)

in which

$R_1$ and $R_3$, which may be identical or different, represent a hydrogen atom, a hydroxyl radical, a linear or branched $C_1$-$C_8$ alkyl radical or a linear or branched $C_1$-$C_8$ alkoxy radical, on the understanding that at least one of the radicals $R_1$ and $R_3$ represents a hydroxyl, alkyl or alkoxy radical, and

$R_2$ and $R_4$, which may be identical or different, represent a hydrogen atom or a hydroxyl radical, on the understanding that at least one of the symbols $R_2$ and $R_4$ represent a hydroxyl radical,

for its use as a medicinal product.

19. Compound of formula (I) according to Claim 18, characterized in that it is selected from 3',5'-di-tert- butyl-4'-hydroxy-3-benzylidene-10-camphorsulphonic acid, 3'-tert-butyl-2'-hydroxy-5'-methoxy-3-benzylidene-10-camphorsulphonic acid, 3'-tert-butyl-2'-hydroxy-5'-methyl-3-benzylidene-10-camphorsulphonic acid and their alkali metal or amine salts.

20. Compound of formula (I) according to Claim 18 or 19, for its use in the treatment of skin allergies and

inflammations.

**21.** Pharmaceutical composition, characterized in that it comprises an effective amount of at least one compound of formula (I) according to Claim 18 or 19, in a non-toxic vehicle or excipient.

**22.** Pharmaceutical composition intended for topical administration, presented in the form of a cream, ointment, pomade, solution, gel, lotion, spray or suspension, characterized in that it contains at least one compound of formula (I) according to Claim 18 or 19.

**23.** Pharmaceutical composition intended for oral administration in the form of tablets, hard gelatin capsules, dragees, syrups, suspensions, solutions or emulsions, characterized in that it contains at least one compound of formula (I) according to Claim 18 or 19.

**24.** Pharmaceutical composition according to Claim 22, characterized in that it contains the active compound of formula (I) in proportions of between 0.25 and 4% by weight relative to the total weight of the composition.

**25.** Use of a compound of formula (I) according to Claim 18 for the preparation of a medicinal product intended for the treatment of skin allergies and inflammations.